(19) Europäisches Patentamt
European Patent Office
Office européen des brevets

(11) EP 1 382 626 A1

(12) **DEMANDE DE BREVET EUROPEEN**

(43) Date de publication:
**21.01.2004 Bulletin 2004/04**

(51) Int Cl.[7]: **C08G 18/80**, C09D 175/02

(21) Numéro de dépôt: **03022382.0**

(22) Date de dépôt: **01.10.1999**

(84) Etats contractants désignés:
**AT BE CH CY DE DK ES FI FR GB GR IE IT LI LU MC NL PT SE**

(30) Priorité: **02.10.1998 FR 9812389**

(62) Numéro(s) de document de la (des) demande(s) initiale(s) en application de l'article 76 CBE:
**99946283.1 / 1 129 120**

(71) Demandeur: **Rhodia Chimie**
**92100 Boulogne Billancourt (FR)**

(72) Inventeur: **Bernard, Jean-Marie**
**69440 Mornant (FR)**

(74) Mandataire: **Colombet, Alain André et al**
**Cabinet Lavoix,**
**2, Place d'Estienne d'Orves**
**75441 Paris Cedex 09 (FR)**

Remarques:
Cette demande a été déposée le 06 - 10 - 2003 comme demande divisionnaire de la demande mentionnée sous le code INID 62.

(54) **Isocyanates modifiés**

(57) L'invention concerne des composition pour application successive ou simultanée comprenant :

a) un (poly)isocyanate modifié stable dont au moins une des fonctions isocyanate est modifiée par un bras fonctionnel réticulant, ou groupe à fonctionnalité réticulante, ladite fonctionnalité réticulante étant un groupement carbonate cyclique ; et
b) un agent réactif à fonction amine primaire ou secondaire.

Procédé de préparation de ces compositions et application à la préparation de revêtements minces non expansés, notamment de peintures ou vernis.

EP 1 382 626 A1

## Description

La présente invention a pour objet de nouveaux dérivés isocyanates modifiés. Elle concerne plus particulièrement des nouveaux dérivés isocyanates comprenant au moins une fonction isocyanate modifiée par un groupe ayant une fonctionnalité réticulante.

Elle concerne également un procédé de préparation de ces dérivés, ainsi que l'utilisation de ces dérivés dans la fabrication de revêtements.

La présente invention a en particulier pour objet des composés mono-, oligo- ou polymériques, porteurs de fonctions isocyanates dont au moins une est modifiée par un groupe à fonctionnalité réticulante, le groupement fonctionnel réticulant étant également appelé bras fonctionnel réticulant. La présente invention concerne plus précisément des polyisocyanates dont au moins un groupement fonctionnel isocyanate est modifié par un groupe tel que défini précédemment.

La présente invention concerne également des procédés d'obtention de ces nouveaux dérivés de polyisocyanates modifiés. Elle vise en outre l'utilisation des dérivés ci-dessus dans des compositions utiles pour la préparation de polymères, notamment de polycondensats et de réticulats issus de la réaction desdits polyisocyanates avec des co-réactifs nucléophiles appropriés. Cette préparation est celle qui est exploitée dans les applications industrielles, telles que les revêtements en tout genre, notamment ceux sur les textiles, les verres, les papiers, les métaux, les matériaux de constructions, et les peintures.

Il est connu de masquer les fonctions isocyanates avec des agents dits « masquants », désignés parfois par « bloquants ».

L'utilité du masquage des fonctions isocyanates (masquage désigné parfois par blocage), voire sa nécessité, s'explique par une réactivité trop élevée à température ambiante, de l'isocyanate vis à vis de certains coréactifs ou vis à vis d'un solvant réactif, ou d'une phase, en général continue, support dans le cas d'émulsions ou de suspensions telles que l'eau. Cette réactivité élevée est souvent très gênante notamment pour certaines applications de polyuréthannes, en particulier dans les peintures, car cela impose un conditionnement et parfois une manipulation séparés du co-monomère isocyanate. Il en découle une mise en oeuvre peu commode.

On appelle classiquement « agent masquant » d'une fonction isocyanate un composé susceptible de masquer cette fonction à basse température pour l'empêcher de réagir avec un groupement et se libérant à température élevée pour restaurer la fonction isocyanate initialement présente.

Le groupe modificateur « réticulant » de la présente invention ne constitue pas un tel groupe, dans la mesure où il ne se libère pas dans les conditions de réticulation de la fonction isocyanate avec laquelle le composé qui le portait a réagi.

En revanche, ce groupe est capable dans les conditions appropriées de libérer un groupement fonctionnel capable à son tour de réagir avec une fonction réactive pour conduire notamment à une réaction de réticulation.

Un objectif de la présente invention est de fournir de nouveaux dérivés isocyanates capables de réagir avec un groupement nucléophile approprié et de fournir des réticulats différents de mousses sans pour autant présenter les inconvénients des groupements isocyanates libres.

Un autre but de la présente invention est de fournir des isocyanates comportant un groupe ou bras fonctionnel réticulant qui ne soient que peu ou pas toxiques.

Un autre objectif de l'invention est de fournir de nouveaux dérivés isocyanates modifiés comportant au moins un groupement fonctionnel réticulant qui soient économiques à préparer.

Un autre objectif de l'invention est de fournir de nouveaux isocyanates comprenant un groupement fonctionnel réticulant, donnant accès à des polymères (ou plutôt à des polycondensats) éventuellement réticulés, qui satisfassent au cahier des charges des applications.

Un autre objectif de l'invention est de fournir un procédé de préparation de polymères et/ou réticulats à partir desdits isocyanates modifiés comprenant un groupement réticulant tel que défini ci-dessus.

On connaît de EP 0 419 114 l'utilisation de carbonates cycliques en tant qu'agents d'expansion de mousses obtenues à partir de polyisocyanates organiques, en particulier de polyisocyanates aromatiques.

Dans les conditions de mise en oeuvre des carbonates cycliques utilisées dans ce document comprenant notamment l'utilisation d'un catalyseur basique, les carbonates réagissent avec le groupe isocyanate des composés polyisocyanates, le cas échéant, en présence d'un polyol, avec libération concomitante de $CO_2$, lequel va provoquer l'expansion de la mousse, le cas échéant, de type polyuréthane.

EP 337 926 décrit de manière large des émulsions aqueuses de polymères linéaires, les polymères étant de nature variée et comprenant au moins deux groupes cyclocarbonates en bout de chaîne destinées à permettre l'allongement de la chaîne par réaction avec un groupe epoxy.

Les travaux des inventeurs ont à présent permis de découvrir de manière surprenante que des carbonates cycliques pouvaient par réaction avec des polyisocyanates aboutir, non pas à une mousse mais à des polyisocyanates modifiés stables portant un groupement carbonate cyclique lesquels, par réaction ultérieure avec un composé portant

un hydrogène réactif, conduisaient à des revêtements pour structures industrielles non expansées, notamment des peintures ou vernis.

En outre, les polyisocyanates ainsi modifiés peuvent par réaction avec une molécule nucléophile réactive conduire à des réactions de réticulation avec consommation d'une fonction alcool.

Les objectifs de l'invention et d'autres qui apparaîtront par la suite sont atteints au moyen d'isocyanates, avantageusement de diisocyanates, de préférence de polyisocyanates modifiés, de formule I suivante :

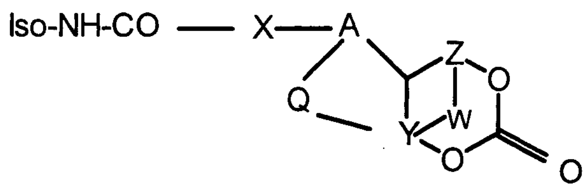

(I)

où :

- Iso est le reste d'un (poly)isocyanate (après transformation d'une fonction isocyanate) ;
- X représente un atome ou un groupement d'atomes susceptible de réagir avec la fonction isocyanate ;
- A représente une liaison ou une chaîne hydrocarbonée linéaire ou ramifiée, ou cyclique, comprenant de 1 à 30, avantageusement de 1 à 18, et de préférence 1 à 5 atomes de carbone ;
- Q représente une liaison, un atome d'oxygène ou de soufre ou une chaîne hydrocarbonée telle que définie pour A ;
- Z représente une liaison ou une chaîne hydrocarbonée telle que définie pour A ;
- Y représente une liaison ou une chaîne hydrocarbonée telle que définie pour A ;

sous réserve que les groupes Z et Y ne représentent pas simultanément une liaison ;

- W représente une liaison, un atome d'oxygène ou de soufre ou une chaîne hydrocarbonée telle que définie pour A ;
- A, Q et W pouvant être identiques ou différents l'un de l'autre.

Les composés tels que définis ci-dessus sont chimiquement stables pendant une durée supérieure à un jour, avantageusement supérieure à une semaine, de préférence supérieure à un mois, plus préférentiellement supérieure à trois mois dans des conditions de stockage habituelles et en l'absence de composés nucléophile réactif.

Par chaîne hydrocarbonée, on entend une chaîne comportant des atomes de carbone et d'hydrogène et éventuellement interrompue par un ou plusieurs hétéroatomes notamment des colonnes IV, V et VI, et en particulier IV A, V A et VI A de la classification périodique des éléments (O, S, Si etc..) ou hétérogroupes (-NH, -N(substitué)) ou substituée par un ou plusieurs groupes choisis notamment parmi aryle, cycloalkyle, hétéroalkyle, halogène, (notamment fluor), une chaîne carbonée linéaire ou ramifiée halogénée (notamment fluorée) ou perhalogénée (notamment perfluorée), un groupe carboxyle, amino-primaire ou secondaire, $NO_2$ et CN.

Pour la définition du tableau périodique, on se reportera au Merck Index, $10^{th}$ Edition (Merck and Co, Rahway, Martha Windholz, Susan Budavari, ed.).

La chaîne hydrocarbonée peut être linéaire ou ramifiée, voire cyclique. Elle peut être saturée ou insaturée.

On peut citer notamment les chaînes alkylène ou particulièrement polyméthylène $-(CH_2)_n-$, n étant généralement compris entre 1 et 12, substituées ou non substituées par des groupes tels que définis ci-dessus.

X peut représenter notamment les fonctions suivantes :

-O,
- S,
=N,
- NR, dans laquelle R représente un atome d'hydrogène ou une chaîne hydrocarbonée ayant généralement de 1 à 12 atomes, de préférence de 1 à 5 atomes de carbone, et interrompue par des hétéroatomes ou hétérogroupes tels que définis précédemment ou portant des substituants tels que définis précédemment.

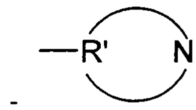

dans laquelle R' représente une chaîne hydrocarbonée telle que définie ci-dessus ayant de 4 à 10 chaînons notamment interrompue par un ou plusieurs hétéroatomes (en particulier des colonnes IV A, V A et VI A de la classification périodique des éléments) tels O, S, Si ou hétérogroupes notamment choisis parmi -N=, -NR- (R étant tel que défini précédemment) et/ou substitué par un ou plusieurs substituants tels que définis précédemment, la chaîne R' formant avec NH un cycle azoté, avantageusement polyazoté, de préférence diazoté, tel le cycle pipérazino,
- CO-NR,
- NR-COO,
- COO,

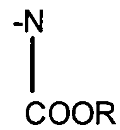
,

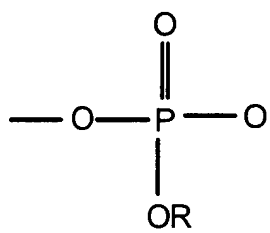
,

-NH-CO-NH,
- N H-CO-NR,
R étant tel que défini précédemment.

Avantageusement, dans les dérivés isocyanates selon l'invention, l'une ou au moins, avantageusement deux, plus particulièrement trois, de préférence toutes parmi les conditions suivantes sont satisfaites :

- X représente l'atome d'oxygène,
- A représente le groupe $-CH_2-$,
- Y représente $-CH_2-$
- Z représente une liaison ou $-CH_2$, de préférence une liaison,
- W et Q représentent une liaison.

Un groupe réticulant préféré est celui obtenu par réaction d'une fonction isocyanate avec le carbonate de glycérol.
Un autre groupe préféré est celui obtenu par réaction d'une fonction isocyanate avec les carbonates d'acide gras ou leurs esters, tels que le 8,9-carbonate de l'acide oléique.
Ainsi que mentionnés ci-dessus, les isocyanates concernés peuvent être des mono-, di-, voire poly-isocyanates.
Parmi les dérivés d'isocyanates modifiés, selon l'invention, figurent :

- les diisocyanates ;
- les composés isocyanates, notamment polyisocyanates comportant un groupe isocyanurate encore appelés trimères ;
- les dérivés isocyanates, notamment polyisocyanates comprenant au moins un groupe urétidinedione, encore appelés dimères ;

- les dérivés isocyanates, notamment polyisocyanates comprenant au moins un groupe biuret ;
- les dérivés isocyanates, notamment polyisocyanates comprenant au moins un groupe carbamate ;
- les dérivés isocyanates, notamment polyisocyanates comprenant au moins un groupe allophanate ;
- les dérivés isocyanates, notamment polyisocyanates comprenant au moins un groupe ester ;
- les dérivés isocyanates, notamment polyisocyanates comprenant au moins un groupe amide ;
- les dérivés isocyanates notamment polyisocyanates comprenant au moins une fonction urée ;
- les dérivés isocyanates notamment polyisocyanates comprenant au moins une fonction imino-cylo-oxa-diazine-dione ;
- les dérivés isocyanates notamment polyisocyanates comprenant au moins une fonction cylo-oxa-diazine-trione ;
- les dérivés isocyanates, notamment polyisocyanates comprenant au moins un groupe isocyanate masqué.
- les dérivés isocyanates, notamment polyisocyanates comprenant une combinaison d'un ou plusieurs des groupes qui viennent d'être mentionnés, en particulier un groupe isocyanurate.

On parle plus généralement d'isocyanates polyfonctionnels tricondensats pour désigner les produits obtenus par (cyclo)condensation, notamment cyclo(trimérisation) d'un ou plusieurs isocyanates monomères identiques ou différents et éventuellement d'un autre monomère.

Plus généralement, ces composés comportent un groupe isocyanurate ou un groupe biuret.

De manière générale, les (poly)isocyanates de l'invention ont un poids moléculaire inférieur à 7500, avantageusement inférieur à 3500, de préférence inférieur à 2500.

Les isocyanates monomères entrant dans la composition des différents composés modifiés énumérés peuvent être aliphatiques, cycloaliphatiques et arylaliphatiques.

Les polyisocyanates modifiés tels que définis ci-dessus peuvent consister en produits de condensation de molécules d'isocyanates identiques ou différents, auquel cas on parlera respectivement d'homo-polyisocyanates et d'hétéropolyisocyanates ou encore en mélanges d'homopolyisocyanates différents et/ou d'hétéropolyisocyanates différents.

Les polyisocyanates préférés visés par l'invention sont ceux dans lesquels au moins une, avantageusement deux, de préférence trois des conditions ci-après sont remplies :

- au moins une, avantageusement deux des fonctions NCO libre(s) ayant réagi avec le groupement réticulant selon l'invention sont reliés à un squelette hydrocarboné, par l'intermédiaire d'un carbone saturé ($sp^3$) ;
- au moins un, avantageusement deux, desdits carbones saturés ($sp^3$) est porteur d'au moins un, avantageusement deux, hydrogène(s), (en d'autres termes, il a été trouvé que l'on obtenait de meilleurs résultats lorsque le carbone porteur de la fonction isocyanate était porteur d'un hydrogène, de préférence de deux hydrogènes) ; il est en outre même préférable que lesdits carbones saturés ($sp^3$) soient au moins pour le tiers, avantageusement au moins pour la moitié, de préférence au moins pour les deux tiers, reliés audit squelette par un atome de carbone lui-même porteur d'au moins un hydrogène, plus préférentiellement deux ;
- tous les carbones par l'intermédiaire desquels les fonctions isocyanates sont reliées au squelette hydrocarboné, soient des carbones saturés ($sp^3$), lesquels sont avantageusement en partie, de préférence en totalité, porteurs d'un hydrogène, de préférence de deux hydrogènes ; il est en outre même préférable que lesdits carbones saturés ($sp^3$) soient au moins pour le tiers, avantageusement au moins pour la moitié, de préférence au moins pour les deux tiers, reliés audit squelette par un atome de carbone lui-même porteur d'au moins un hydrogène, plus préférentiellement deux.

Conformément à une modalité avantageuse de l'invention, les polyisocyanates dont les fonctions NCO sont modifiées par un groupe réticulant tel que défini, sont choisis parmi les produits d'homo- ou d'hétéro-condensation d'alcoylènediisocyanate, comprenant notamment des produits du type "BIURET" et du type "TRIMERES", voire "PREPOLYMERES" à fonction isocyanates comportant notamment des fonctions urée, uréthane, allophanate, ester, amide, et parmi les mélanges en contenant.

Il peut s'agir, par exemple, des polyisocyanates commercialisés par la Société demanderesse sous la dénomination "TOLONATE®".

De manière générale, les polyisocyanates préférés sont les produits d'homo- ou d'hétéro-condensation des isocyanates monomères suivants :

- les polyméthylènediisocyanates et notamment le 1,6-hexaméthylène diisocyanate, le 2-méthyl 1,5-pentaméthylène diisocyanate, le 2,4,4-triméthyl 1,6-hexaméthylène diisocyanate, le 3,5,5-triméthyl 1,6 hexaméthylène diisocyanate, le 1,12-dodécane diisocyanate, l'isocyanato (4)-méthyl 1,8-octylène diisocyanate (TTI ou NTI) ;
- les cyclobutane 1,3-diisocyanate, cyclohexane 1,2-, 1,3- ou 1,4-diisocyanate, le 3,3,5-triméthyl-1 isocyanato-5-isocyanatométhyl cyclohexane (isophorone diisocyanate), IPDI), le bis-isocyanato méthyl norbornane (NBDI), le 1,3-bis(isocyanatométhyl)cyclohexane (BIC), le $H_{12}$-MDI et le cyclohexyl 1,4-diisocyanate ;

- les arylènedialcoylènediisocyanàtes, tel que $OCN-CH_2-\varnothing-CH_2-NCO$ ; ou encore aromatiques tels que le toluylène diisocyanate.

Les isocyanates aromatiques ne sont pas préférés.

Lorsque les polyisocyanates sont relativement lourds, c'est-à-dire qu'ils comportent au moins quatre fonctions isocyanates, au moins une fonction isocyanate étant avantageusement modifiée par un groupement réticulant tel que défini ci-dessus, ou bien lorsque l'on se trouve en face d'un mélange de plusieurs composés porteurs de fonction(s) isocyanate(s), les premières conditions deviennent :

- au moins un tiers, avantageusement deux tiers, de préférence quatre cinquième des fonctions NCO libres ou comportant un groupement réticulant tel que défini ci-dessus, sont reliées à un squelette hydrocarboné, par l'intermédiaire d'un carbone saturé ($sp^3$).
- au moins un tiers, avantageusement deux tiers, de préférence quatre cinquième desdits carbones saturés ($sp^3$) est porteur d'au moins un, avantageusement deux hydrogène(s) (en d'autres termes, il a été trouvé que l'on obtenait de meilleurs résultats lorsque le carbone porteur de la fonction isocyanate était porteur d'un hydrogène, de préférence de deux hydrogènes).

Il est en outre même préférable que lesdits carbones saturés ($sp^3$) soit au moins pour le tiers, avantageusement au moins pour la moitié, de préférence au moins pour les deux tiers, reliés audit squelette par un atome de carbone lui-même porteur d'au moins un hydrogène, avantageusement deux.

Sont particulièrement visés les mélanges pour lesquels la (quasi)totalité des fonctions isocyanates libres ou comportant un groupement réticulant tel que défini ci-dessus, réponde aux critères ci-dessus.

Les fonctions isocyanates non modifiées selon la présente invention, peuvent être soit libres, soit masquées par un groupe masquant thermolabile usuel.

On appelle agent masquant au sens de l'invention un groupe réagissant avec une fonction isocyanate telle que le composé isocyanate masqué montre à une température d'au moins 50°C, avantageusement au moins 60°C, et de préférence au moins 70°C et d'au plus 350, avantageusement d'au moins 250°C, de préférence d'au plus 200°C, les températures les plus élevées étant réservées à des procédés de réticulation flash et après une période de chauffage comprise entre quelques secondes et quelques heures, avantageusement 10 secondes et 20 minutes, une "libération" du groupe masquant au moins égale à 50 %, dans le test à l'octanol, dont le mode opératoire est décrit plus loin.

En général, on considère comme composés isocyanates masqués tout composé qui conduit à la libération de l'agent masquant avec régénération de la liaison isocyanate ou transformation de celle-ci en une liaison uréthane si il y a présence d'un alcool aliphatique primaire ou secondaire ou en une liaison urée si il y a présence d'une fonction amine aliphatique primaire ou secondaire.

En présence de catalyseurs actifs ou latents activables thermiquement ou par oxydation, les cinétiques de libération sont accélérées ou les températures de régénération de la fonction isocyanate (par libération du groupe masquant) sont abaissées.

A titre d'exemples, lorsque le groupe masquant est l'imidazole, le test à l'octanol donne un taux de libération de 50 % avec formation respective de 50 % de carbamate d'octyle correspondant à 80°C et de 100 % à 100°C, la 2-hydroxy-pyridine donnant des taux respectifs de 90 % et 100 % à ces températures.

En présence de 0,1 % en poids de dibutyl étain, le taux de déblocage de l'imidazole à 80°C est augmenté à 90 %.

On peut citer notamment les groupes masquants de type hétérocycle (poly)azoté, tels que l'imidazole, le pyrazole, le 1,2,3-triazole ou le 1,2,4-triazole, lesdits hétérocycles pouvant éventuellement porter des susbstituants ; ou encore les lactames, les phénols éventuellement substitués tels que les parahydroxybenzoates et les oximes, notamment la méthyléthylcetoxime (MEKO), l'oxime du pyruvate de méthyle, l'oxime du pyruvate d'éthyle (POME) et la cyclohexanone oxime.

Les groupes masquants comportent éventuellement des fonctions ioniques acides telles que des fonctions acides carboxyliques, sulfoniques ou des fonctions ioniques basiques telles que des fonctions amines tertiaires. Ces groupements ioniques présentent un intérêt tout particulier car ils facilitent la réalisation de certains types de formulations telles que l'obtention de poudres, de dispersions ou de solutions aqueuses.

Dans un mode de réalisation avantageux, les polyisocyanates ou plus exactement la composition polyisocyanate de l'invention comprend au moins deux groupes masquants différents choisis de manière que dans le test à l'octanol à 110°C, le rapport

$$D = \frac{\text{pourcentage d'agent masquant se débloquant en premier à } 110°C}{\text{pourcentage d'agent masquant se débloquant en dernier à } 110°C}$$

soit supérieur à 4/3, avantageusement supérieur à 1,5, de préférence supérieur à 2.

Les groupes masquants peuvent être notamment une oxime et le triazole (1,2,3-triazole ou 1,2,4-triazole), l'oxime étant avantageusement la méthyl-éthyl cétoxime, la méthyl-amyl-cétoxime, l'oxime du pyruvate de méthyle ou l'oxime du pyruvate d'éthyle.

Les dérivés isocyanates modifiés selon l'invention se présentent sous forme liquide ou sous forme de poudre.

Les dérivés isocyanates modifiés selon l'invention peuvent être préparés en mettant en oeuvre des procédures bien connues de l'homme du métier par condensation d'un composé de formule générale II :

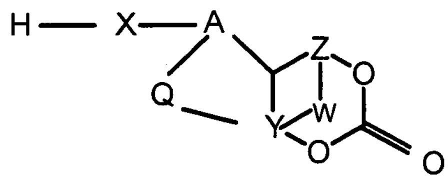

(II)

dans laquelle X, A, Q, Y, Z et W ont les mêmes spécifications que précédemment, avec un isocyanate.

Le cas échéant, les fonctions réactives du composé de formule générale II autres que XH sont protégées avec un groupement protecteur approprié et ultérieurement déprotégées.

Ainsi, les isocyanates modifiés selon l'invention peuvent être obtenus :

- lorsque X représente un atome d'oxygène ou de soufre, par condensation des composés isocyanates que l'on souhaite modifier avec le composé de formule générale I telle que définie précédemment dans laquelle X représente O ou S, sous chauffage, éventuellement en présence d'un catalyseur en présence ou en absence de solvant, le solvant pouvant notamment être un ester, un éther, un hydrocarbure aromatique.
- lorsque X représente un groupe NR tel que défini ci-dessus par condensation avec un composé isocyanate à la température ambiante ou sous chauffage dans un solvant usuel.

Le cas échéant, les isocyanates sont mis à réagir avant ou après réaction avec un composé de formule générale II tel que défini ci-dessus, avec un agent masquant dans les conditions de réaction appropriées.

On peut également faire réagir les isocyanates sur un mélange de composés de formule générale II et d'agents masquants dans les conditions de réaction appropriées, connues de l'homme du métier.

On peut également préparer les composés de l'invention en mélangeant les composés isocyanates avec des composés précurseurs de la molécule (II) tels que les diols vicinaux et des agents de carbonylation activés, les molécules activant le carbonyle, libérées après réaction de formation du carbonate, pouvant servir le cas échéant d'agents de masquage de la fonction isocyanate. On peut ainsi citer à titre d'exemples les composés carbonyles activés tels que le carbonyldiimidazole, le carbonyl-di-1,2,4-triazole, le carbonyl di-méthyl-éthyl-cétoxime, le N,N'-disuccinimidylcarbonate. Les composés libérés après réaction avec le diol et formation du carbonate tels que l'imidazole, le 1,2,4-triazole, la méthyl-éthyl-cétoxime sont connus comme des agents masquants des fonctions isocyanates.

Un groupe d'isocyanates modifiés préféré selon l'invention est constitué par les dérivés diisocyanates tels que mentionnés ci-dessus comportant au moins une partie des fonctions isocyanates de préférence au moins 100 à 1 %, avantageusement 100 à 30 %, en poids, modifiées par un groupe réticulant tel que défini ci-dessus au moins 1 %, avantageusement au moins 5 %, et de préférence 10 %, et jusqu'à 99 %, avantageusement jusqu'à 95%, et de préférence jusqu'à 70 %, en poids des fonctions isocyanates, modifiées par un groupe masquant tel que défini ci-dessus.

Ces dérivés isocyanates ont des applications notamment sous forme de poudres ou peuvent être utilisés en milieu aqueux.

Les compositions polyisocyanates objets de la présente invention peuvent être constituées par un mélange comprenant au moins 1%, et au plus 99%, de préférence au moins 10 % et au plus 90% d'un polyisocyanate portant majoritairement le groupe réticulant de l'invention et au moins 1 %, et au plus 99%, de préférence au moins 10 % et au plus 90% d'un autre polyisocyanate portant majoritairement un groupe réticulant et/ou une autre molécule dérivé d'un diisocyanate porteuse de fonctions isocyanates libres et/ou masquées et ne comportant pas de groupe réticulant.

De manière particulièrement avantageuse, le groupe réticulant et le groupe NCO libre et/ou masqué sont portés par la même molécule (poly)isocyanate.

De préférence, la composition (poly)isocyanate de l'invention ne comprend pas de groupes carboxyliques

portés par une molécule de formule I telle que définie précédemment.

Cependant, les agents masquants des fonctions isocyanates peuvent porter des groupes ioniques et en particulier des groupes carboxyliques ou amines tertiaires, ces groupes ioniques pouvant être salifiés en partie ou dans leur totalité.

Les composés peuvent garder des fonctions isocyanates libres, notamment au moins 1 %, avantageusement au moins 5 % et de préférence au moins 10 %, et jusqu'à 99 %, avantageusement jusqu'à 70 %, en poids.

Un second groupe d'isocyanates modifiés préféré selon l'invention est constitué par les mélanges d'isocyanates polyfonctionnels tricondensats, de préférence vrais (issus de la (cyclo)trimérisation théorique de trois molécules monomères isocyanates et éventuellement autres monomères et comportant un cycle isocyanurate et/ou biuret) et d'allophanates, et/ou dimères et/ou urées, uréthanes, biurets, carbamates comportant au moins une partie de préférence au moins 1 à 100 %, avantageusement 30 à 100 % en poids, des fonctions isocyanates modifiées par un groupe réticulant tel que défini ci-dessus.

Les composés peuvent garder des fonctions isocyanates libres, notamment de 1 à 99 %, avantageusement 5 à 70 %, en poids.

Un troisième groupe de composés préféré est constitué par les mélanges physiques de plusieurs isocyanates polyfonctionnels tricondensats, avec des allophanates, urétidinediones, dimères, comportant de 100 à 1, avantageusement de 70 à 1 %, en poids, de groupes isocyanates modifiés par un groupe réticulant selon l'invention et de 1 à 99 %, avantageusement de 5 à 70 % en poids de fonctions isocyanates masquées par un groupe masquant, tel que défini précédemment.

Un quatrième groupe de dérivés isocyanates modifiés selon l'invention est constitué par les isocyanates modifiés comportant des groupes isocyanates libres et/ou des groupes isocyanates masqués ainsi que des groupes allophanates et/ou urétidinedione.

Les composés peuvent garder des fonctions isocyanates libres, notamment de 1 à 99 %, avantageusement 5 à 70 %, en poids.

Dans les mélanges mentionnés ci-dessus, les divers composés polyfonctionnels peuvent être issus de la polycondensation de plusieurs monomères identiques ou différents.

Dans le cas des mélanges d'isocyanates polyfonctionnels, les différents isocyanates polyfonctionnels peuvent être obtenus à partir d'isocyanates différents ou de mélange d'isocyanates différents.

On peut par exemple utiliser un mélange d'isocyanates d'HDI et d'allophanates de butyle et d'HMDI.

On peut aussi, pour préparer les composés de l'invention, utiliser les mélanges bruts de (cyclo)condensation des isocyanates de départ sur eux-mêmes (dimérisation, trimérisation,..) avant élimination ou après élimination partielle du ou des monomères isocyanates de départ n'ayant pas réagi. Ces mélanges bruts se caractérisent par le fait que le pourcentage pondéral du ou des monomères isocyanates de départ rapportés à l'ensemble des produits du mélange est compris entre 1 et 95 %, de préférence entre 5 et 80 %.

De même, on peut utiliser des mélanges dérivés de ces mélanges bruts de (cyclo)condensation des isocyanates. Par mélange dérivé, on entend le produit de la réaction des composés du mélange brut avec des composés nucléophiles ou portant des fonctions hydroxyles, sulfhydriles ou amines capables de réagir avec les fonctions isocyanates des composés du mélange.

En général, les compositions d'isocyanates utilisées sont des mélanges de diverses molécules issues de polymérisations ou de polycondensation, auquel cas ce qui vient d'être expliqué sur ce qui est préféré ci-dessus s'applique avec des valeurs fractionnaires et statistiques.

Les fonctions isocyanates libres ou libérées par départ du groupe peuvent former par condensation avec des groupements à hydrogène mobile, notamment des polyols, ou des polyamines ou des polysulfhydryles, des prépolymères à fonctions carbonates pendantes et fonctions terminales isocyanates libres ou alcools ou amines ou sulfhydryles selon le rapport NCO/XH, X étant tel que défini ci-dessus.

Après ouverture du groupe réticulant avec un agent réactif approprié, le prépolymère ainsi obtenu peut être ultérieurement réticulé.

Les fonctions réagissant avec le groupement réticulant selon l'invention sont les fonctions alcools, amines primaires ou secondaires, des composés hétérocycliques azotés possédant un atome d'hydrogène réactif, des oximes ou des phénols, de préférence des phénates ou des carboxylates. De préférence, on choisira l'ammoniaque, les amines primaires ou secondaires ou les hétérocycles azotés, par exemple les guanidines ou leurs sels qui agissent par ouverture du cycle.

Pour obtenir des réseaux ou films polyuréthannes, on peut faire réagir ces prépolymères avec des amines de préférence di- ou poly-amines, primaires ou secondaires de préférence. On obtient ainsi de réseaux à fonctions hydroxyles pendantes qui peuvent être soit autoréticulées avec les NCO présents dans le milieu, soit autorisent un greffage ou permettent une réaction de réticulation avec des mélanges réactifs avec ces fonctions.

De même, on peut faire réagir ces produits porteurs de fonctions isocyanates libres et carbonates avec des amines pour donner des réseaux polyurées uréthannes à fonctions hydroxyles et/ou carbonates pendantes. Si la

quantité de fonctions isocyanates est supérieure à la quantité d'amines, alors les fonctions alcools libérées par ouverture du cycle carbonate peuvent réagir avec les fonctions isocyanates en excès. La vitesse d'ouverture du cycle carbonate est fonction de l'amine et de la réactivité de l'isocyanate avec cette amine.

Dans certains cas, l'amine réagira préférentiellement avec la fonction carbonate avant la fonction isocyanate. On aura donc la possibilité d'avoir une réaction de la fonction isocyanate avec les fonctions alcools libérées.

La réaction d'ouverture du cycle avec une amine conduit à la génération d'une liaison carbamate en libérant une fonction OH libre (de type alcool primaire uniquement, dans le composé de formule générale I, Z est différent d'une liaison et de type alcool primaire et/ou alcool secondaire si dans le composé de formule générale I, Z est une liaison notamment dans le cas du carbonate de glycérol).

Dans certains cas, la fonction alcool libérée peut être tertiaire mais dans ce cas, sa réactivité est faible et elle n'est pas préférée.

La fonction OH libre peut à son tour réagir à une température donnée avec une fonction isocyanate, éventuellement bloquée avec départ du groupe bloquant pour conduire à un réseau de manière rapide, notamment lorsque les groupes isocyanates libres ou bloqués sont portés par un isocyanate polyfonctionnel, un trimère, un dimère, un prépolymère.

La fonction hydroxyle libérée peut également réagir avec d'autres composés qui peuvent être présents dans une formulation utilisant les composés de l'invention. On peut ainsi citer à titre d'exemple les anhydrides d'acides ou les composés acides qui peuvent réagir avec la fonction hydroxyle libérée pour donner un ester ou ester acide.

Les fonctions isocyanates libres ou libérées par départ du groupe bloquant peuvent réagir avec tout type de composés à hydrogène mobile, notamment les alcools, thiols, uréthannes, etc, selon la température de réticulation utilisée.

Une autre possibilité intéressante consiste à ouvrir le cycle du groupe réticulant par une amine polyalcoxylée, notamment polyéthoxylée de manière à obtenir un produit de condensation présentant de bonnes propriétés émulsifiantes. Ce type de composé est particulièrement intéressant dans le cadre de réactions ultérieures de polymérisation en émulsion, notamment par réaction avec un groupe isocyanate libre ou libéré par départ d'un groupe bloquant.

Les sels, de préférence d'amine, de préférence d'acides faibles réagissent également avec les fonctions isocyanates et/ou carbamates en fonction de la température imposée par la réticulation.

Pour ce qui est des sels, on choisira, de préférence, un sel d'acide faible de pKa supérieur à 2,5 si la température de réticulation est inférieure à 80°C. Pour des températures de réticulation supérieures, les sels d'acides de pka inférieurs à 3 peuvent être utilisés. On peut aussi introduire un composé capable de neutraliser le sel de l'amine réactive par une base capable de faire l'échange d'ions et libérer l'amine qui, parce que nucléophile, est alors capable d'ouvrir le cycle. Comme base, on peut citer les hydroxydes métalliques (hydroxyde de sodium, de potassium...), les amines tertiaires 5-triéthylamine trio-cylamine, N,N-diméthylaminoéthanol...), les alcoolates métaliques (méthylate de sodium...), les sels alcalins d'acides faibles (acétate de sodium, hydrogéno-carbonate de sodium, carbonate de potassium ...).

Les sels d'amine présentent l'avantage d'avoir un "pot-life" amélioré du fait de la diminution de réactivité de l'amine correspondante.

Au cas où l'on fait réagir des composés isocyanates modifiés selon l'invention comportant également des groupes isocyanates libres, on veillera à choisir les isocyanates de manière à favoriser soit la réaction de l'amine avec l'isocyanate, soit la réaction de l'amine avec le carbonate. On pourra augmenter cette sélectivité en jouant sur la température, le catalyseur ou l'encombrement stérique de l'amine et/ou de l'isocyanate.

Il est également avantageux d'utiliser des composés précurseurs d'amines qui peuvent restaurer l'amine par un processus chimique ou physique tels que par exemple les imines, les oxazolines, les oxazolidines, qui par hydrolyse libèrent l'amine qui peut alors ouvrir le cycle carbonate.

Il convient de citer le cas particulier des molécules isocyanates qui constituent également des formes masquées d'amines et qui peuvent, par hydrolyse, restaurer la fonction amine. Ces isocyanates peuvent être de simples isocyanates (une seule fonction isocyanate par molécule).

On choisira avantageusement l'IPDI.

Les produits de l'invention porteurs de fonctions isocyanates éventuellement masquées et carbonates peuvent également être utilisés tels quels.

En fonction du taux de $CO_2$ et de la cinétique de réticulation, on obtient de manière surprenante des revêtements non expansés, notamment des revêtements de type peintures ou vernis d'une épaisseur du film qui n'est pas supérieure à 100 µm et qui peuvent présenter un aspect mat, satiné ou brillant selon les conditions de mise en oeuvre.

Les isocyanates modifiés selon l'invention conduisent à une température élevée supérieure à 100°C à des composés oxétanes avec libération de $CO_2$. Ces composés oxétanes peuvent être utilisés comme agents réticulants avec des polyols ou des polyamines, de préférence des polyols, *in situ* dans le film.

Après ouverture du carbonate avec une amine, on obtient des dérivés possédant au moins deux fonctions alcools qui peuvent être utilisées pour préparer des polymères ou pour apporter des propriétés particulières à un

polymère par exemple, le rendre autoémulsifiable si on a ouvert les fonctions carbonates par une amine polyoxyéthylénée ou conduire à un revêtement "anti-graffitis" ou "mar-résistance" si on a ouvert les fonctions carbonates par une amine silicone ou perfluorée.

Après ouverture du cycle carbonate par l'eau ou les solutions aqueuses basiques, les dérivés d'isocyanates selon l'invention peuvent conduire également à des polyols tétrafonctionnels qui peuvent être utilisés comme agents réticulants, notamment du fait de la double réactivité due à la présence de fonctions alcools primaires et secondaires.

De manière générale, les dérivés isocyanates modifiés selon la présente invention présentent l'avantage d'une réactivité élevée et contrôlée et d'un pouvoir réticulant élevé, propriétés qui sont obtenues par addition d'un bras n'apportant qu'un faible accroissement du poids moléculaire de l'isocyanate de départ.

De même, les dérivés polyisocyanates à fonctions uréthanes-carbonates pendantes peuvent conduire à la formation d'allophanates à fonctions carbonates pendantes.

L'un des nombreux intérêts des nouveaux polyisocyanates selon l'invention est qu'ils peuvent servir de base à la préparation de polymères et/ou de réticulats utiles, par exemple, comme constituants principaux de revêtements en tous genres, tels que des peintures. Dans de telles utilisations, les qualités de dureté des polymères réticulables font partie de celles qui sont recherchées sur le plan technique et fonctionnelles.

Dans le cas des formulations aqueuses, les polyisocyanates à fonction réticulante de l'invention peuvent être mis en émulsion au moyen de divers composés tels que des tensioactifs, ou des polyols à caractère émulsionnable ou être rendus hydrosolubles par greffage de fonctions non ioniques telles que polyoxyde d'alkylène ou de fonctions ioniques acides telles que celles des acides parahydroxybenzoïque (PHBA) diméthylolpropionique, sulfamique et des dérivés phosphoriques ou des fonctions ioniques basiques telles que les N,N-dialkyl hydroxylalkylamines, notamment la N,N-diméthyl éthanolamine ou les dérivés des guanidines.

Le greffage peut être réalisé de manière réversible (PHBA) ou irréversible.

L'invention sera mieux comprise, ses variantes, ainsi que d'autres de ses avantages ressortiront dans les exemples qui suivent.

## TEST A l'OCTANOL

Mode opératoire :

Dans un tube type Schott avec agitation magnétique, on charge environ 5 mmol en équivalent NCO masqué protégé à évaluer.

On ajoute 2,5 à 3 ml de dichloro-1,2 benzène (solvant) l'équivalent d'octanol-1 (5 mmol, soit 0,61 g et éventuellement avec le catalyseur à tester avec le groupe masquant).

Le milieu réactionnel est ensuite porté à la température testée. On chauffe alors pendant une durée donnée en général six heures, sauf indications contraires, à la température testée, de façon à débloquer et ainsi rendre réactives les fonctions isocyanates. La réaction terminée, le solvant est éliminé par distillation sous vide, et le résidu est analysé en RMN, masse et infrarouge.

A partir de ces données, on évalue le pourcentage de fonction isocyanate masquée condensée avec l'octanol-1 et donc le pourcentage d'agent masquant libéré.

## EXEMPLES

### EXEMPLE 1 : Synthèse de l'allophanate de HDI et de butyle

Dans un réacteur tricol de 6 l, on introduit 4 787 g de HDI. 527,1 g de butanol 1 sont ajoutés en 45 minutes. Le milieu réactionnel est chauffé de telle manière que la température obtenue après 45 minutes suivant le début d'addition du butanol, soit de 125°C. Environ 1,3 g de dibutyl dilaurate d'étain est alors ajouté et la température du mélange réactionnel est montée à 140°C.

Après 5 heures de réaction, le titre NCO mesuré est de 0,786 contre 1,19 pour l'HDI de départ. L'HDI en excès est éliminé par deux distillations successives sous vide de 0,5 mm d'Hg, à 140°C avec un débit compris entre 400 et 1 000 g/heure.

Le produit distillé présente un taux de NCO de 0,405 soit 17 % en poids, une viscosité à 25°C de 140 mPa. $s^{-1}$. Le taux de HDI est de 0,4 %.

### EXEMPLE 2 : Synthèse d'un allophanate de HDI et de n-butyle masqué par le triazole et le carbonate de glycérol

Dans un réacteur double enveloppe, on introduit successivement :

- 350 g d'allophanate de hexaméthylène diisocyanate et de butyle de l'exemple 1 dont le taux de fonction isocyanate est de 0,405 soit 17 % en poids de fonction NCO pour 100 g de produit ; et
- 51,4 g de 1,2,4-triazole.

On chauffe le mélange réactionnel de telle manière qu'il atteint 113°C en 20 minutes. Le 1,2,4-triazole est alors consommé entièrement.

À ce moment, on ajoute 83,6 g de carbonate de glycérol et 3 g de triéthylamine. Une exothermie a lieu qui fait monter la température à 131°C. La réaction est alors maintenue à 110°C pendant 3 heures environ.

Par analyse infrarouge, on constate que les fonctions isocyanates libres sont pratiquement négligeables ce qui indique une réaction pratiquement complète de ces fonctions.

Le produit est ensuite coulé dans un récipient et laissé refroidir (488 g).

Le produit froid est un liquide visqueux qui ne coule pas indiquant une viscosité supérieure à 10 000 mPa. $s^{-1}$ à 25°C.

**EXEMPLE 3: Synthèse d'un polyisocyanate à fonctions isocyanurates dont une partie des fonctions isocyanates est masquée par le triazole et une autre partie des fonctions isocyanates est modifiée par le carbonate de glycérol**

Dans un réacteur tricol, on introduit successivement :

- 350 g de HDT (trimère de l'HDI) dont le taux de fonctions isocyanates est de 0,525, soit 22 % en poids ;
- 63,2 g de 1,2,4- triazole ;
- et après 25 minutes de réaction, 1,5 g de triéthylamine.

Le mélange est chauffé dès l'addition du 1,2,4-triazole. Le mélange atteint la température de 99°C après 45 minutes.

À ce moment, on ajoute 108 g de carbonate de glycérol et 1,5 g de triéthylamine.

La température du mélange est portée à 123°C puis celui-ci est laissé en réaction pendant une heure environ jusqu'à ce que le titre en fonctions isocyanates (NCO) libres soit pratiquement nul.

Le produit est soutiré puis laissé à refroidir (produit obtenu : 524,2 théorique, 517 g mesuré). Le produit froid est un solide qui est ensuite broyé et dont le taux de fonctions isocyanates potentiel est de 7,35 % en poids et le taux de fonctions carbonates (-O-C(O)-O) de 10,48 % en poids. Le rapport molaire NCO modifié/O-C(O)-O est de 1.

**EXEMPLE 4 Synthèse d'un polyisocyanate à fonctions isocyanurates dont les fonctions isocyanates sont modifiées par le carbonate de glycérol.**

Dans un ballon de 500 ml, on introduit 300 g de Tolonate® HDT, 184,4 g de carbonate de glycérol puis on chauffe le milieu réactionnel à 86°C. Le produit est ensuite soutiré et après refroidissement, broyé pour donner une poudre dont le titre en NCO est de 0,001 et le taux de fonctions carbonates (-O-C(O)-O) de 19,3 % en poids, soit 0,3 mole de fonctions carbonates (-O-C(O)-O-) pour 100 g de produit.

L'analyse RMN 1H indique la présence de 54 % de trimère (incluant du dimère) modifié, 8,5 % de biuret modifié et 0,39 % de carbonate de glycérol libre. Les bandes caractéristiques des produits en infrarouge sont les suivantes :

CO carbonate : 1798 $cm^{-1}$
CO carbamate : 1721 $cm^{-1}$
CO trimère isocyanurate : 1685 $cm^{-1}$
Cycle isocyanurate : 1468 $cm^{-1}$
CO-NH carbamate : 1531 $cm^{-1}$
NH carbamate : 3362 $cm^{-1}$

en milieu $CH_2Cl_2$ les bandes allophanates sont observées : NH allophanate : 3369 $cm^{-1}$/NH carbamate : 3444 $cm^{-1}$.

**EXEMPLE 5 : Synthèse d'HDI modifié par le carbonate de glycérol**

On procède de la même manière que pour l'exemple 3. La quantité d'HDI est de 168 g et la quantité de carbonate de glycérol est de 236 g.

Après cinq heures à 80°C, le taux de NCO résiduel est de 0,011. Deux heures supplémentaires à 100°C donnent après soutirage et refroidissement un composé en poudre dont le titre en fonctions NCO libres est de 0,001 et le titre en fonctions carbonates de 44,5 %.

**EXEMPLE 6 :**

**Synthèse du monoester de carbonate de glycérol de l'acide succinique**

Dans un réacteur sous agitation et placé sous atmosphère inerte (courant d'azote), on charge successivement 30 ml de toluène, 60,6 g de carbonate de glycérol et 51,3 g d'anhydride succinique.
Le milieu réactionnel est chauffé à 90°C pendant 6 heures et à 120°C pendant 2 heures puis maintenu sous agitation pendant 2 heures.
On procède ensuite à une extraction liquide du milieu réactionnel froid dans une ampoule à décanter. On ajoute 500 ml d'une solution aqueuse bicarbonatée 0,5 M froide (10°C), puis 200 ml d'acétate d'éthyle. La phase organique est soutirée et relavée deux fois avec une même solution aqueuse bicarbonatée 0,5 M froide (10°C).
Les phases aqueuses sont alors réunies, puis acidifiées par une solution aqueuse HCl 1 M jusqu'à ce que le pH de la solution soit acide ( pH = 2). La phase aqueuse est alors extraite trois fois avec 500 ml d'acétate d'éthyle. Les phases organiques sont ensuite séchées sur du sulfate de sodium sec. Après filtration, le solvant est concentré sous vide pour conduire à un solide blanc.
Le rendement de réaction est de 27 % (rendement non optimisé).
L'analyse RMN du produit indique que le produit présente les bandes caractéristiques suivantes :

- RMN $^1H$ (DMSO): $CH_2$ succinique à 2,43 et 2,65 / $CH_2$ en alpha de l'oxygène de la fonction ester 4,16 - 4,34 / $CH_2$ en alpha du carbonate 4,16 - 4,34 / CH en alpha du carbonate 5,01.
- RMN $^{13}C$(DMSO): $CH_2$ succinique à 28,7 / C=O de la fonction acide à 173,4 / C=O de la fonction ester à 172,0 / C=O de la fonction carbonate à 154,8 / $CH_2$ en alpha de l'oxygène de la fonction ester 63,4 / $CH_2$ en alpha du carbonate 66,0 / CH en alpha du carbonate 74,4.

La structure du produit est également confirmée par Analyse Infra rouge. Le point de fusion du produit est de 102-103°C (Banc Kofler).

**EXEMPLE 7 :**

**Synthèse d'une résine à fonctions carbonates pendantes et à motifs essentiellement isocyanurates obtenue par réaction du composé** de **l'exemple 6 et d'HDT**

L'HDT utilisé est du Tolonate® HDT, commercialisé par la société RHODIA, comprenant un mélange de composés de cyclopolycondensation de l'hexaméthylène diisocyanate (HDI) sur lui même ayant la composition suivante :

| ***Produits*** | ***Tolonate® HDT*** | ***Tolonate® HDT-LV2*** |
|---|---|---|
| HDI | 0,2% | 0,24% |
| Monocarbamate de butyle | 0,5% | 0,5 % |
| Dimère vrai de HDI | 2,5% | 14,2 % |
| Trimère vrai de HDI | 50,1 % | 56 % |
| Bis trimère + Trimère dimère | 24 % | 20,6 % |
| Lourds | 18,7% | 7,6 % |
| Biuret | 4,0 % | 0,86 % |

Les trimères vrais sont constitués de trois chaînes HDI cyclocondensées sur elles-mêmes en un cycle isocyanurate.
Les dimères vrais sont constitués de deux chaînes HDI cyclocondensées en un cycle uretidine-dione.
Les oligomères trimères plus lourds sont constitués de plus de trois chaînes d'HDI cyclocondensées et de plus d'un cycle isocyanurate. Le TOLONATE® HDT possède des fonctions isocyanates libres à raison de 22 % en

poids, généralement de fonctions NCO pour 100 g de produit.

On procède comme suit :

Dans un réacteur équipé d'un agitateur et placé sous atmosphère inerte d'azote, on introduit 14,77 g de Tolonate® HDT dont le titre mesuré par la méthode de dosage en retour à la dibutylamine est de 0,518 mole de fonction NCO pour 100 g de produit. Une même quantité molaire (ratio molaire NCO / COOH = 1) du monoester de carbonate de glycérol de l'acide succinique (obtenu comme décrit dans l'exemple ci dessus) est ajoutée au mélange réactionnel. On ajoute de la triéthylamine (NEt3) à raison de 1 % molaire par rapport aux fonctions carboxyliques du monoester (ratio molaire NEt3/ COOH = 0,01). Le mélange est alors chauffé et agité pendant cinq heures à 110°C. On observe un dégagement gazeux de dioxyde de carbone. Le produit est récupéré.

Le produit obtenu est bien le produit de réaction attendu et présente les bandes caractéristiques majoritaires suivantes en infrarouge :

Très faible bande NCO à 2257 $cm^{-1}$
Bande C=O carbonate à 1795 $cm^{-1}$
Bande C=O ester succinate à 1739 $cm^{-1}$
Bandes isocyanurates de l'HDT à 1685 et 1466 $cm^{-1}$
Bande -C=O-NH- amide à 1546 $cm^{-1}$ et Bande C=O amide secondaire à 1640 $cm^{-1}$
Bande NH à 3350 - 3250 $cm^{-1}$

## EXEMPLE 8 :

**Synthèse d'une résine à fonctions carbonates pendantes et à motifs essentiellement isocyanurates et urétidines diones obtenue par réaction du composé de l'exemple 6 et avec une composition d'HDT comprenant de l'HDI dimère (Tolonate® HDT).**

La composition d'HDT Tolonate® est celle du tableau de l'exemple 7. Elle est obtenue comme suit :

Dans un réacteur de 1 litre muni d'une colonne réfrigérante et chauffé au moyen d'un bain d'huile, on introduit sous agitation 1 000 g d'HDI.

Le milieu réactionnel est chauffé pendant 1 heure 30 à 160°C. On ajoute ensuite 10 g (1 % en poids) d'HMDZ (hexaméthyldisilazane ). Le milieu réactionnel est chauffé pendant 30 minutes à 140°C puis refroidi. Lorsque la température atteint 88°C, 5,5 g de n-butanol sont ajoutés. Après une heure de réaction, le produit est purifié par distillation sous vide.

Les lourds sont comptabilisés en tris-trimère. Le massif bis-trimère (composé majoritaire) comprend des tétramères (trimère-dimère) et de l'imino-trimère.

La composition résultante présente une viscosité à 25°C de 509 cps (509 mPa.s).

Le Tolonate® HDT LV2 possède des fonctions isocyanates libres à raison de 0,544 mole de fonction NCO pour 100 g de produit Tolonate® HDT LV2.

On procède ensuite comme pour l'exemple précédent en travaillant sur 14,8 g de Tolonate® HDT LV2.

On utilise les mêmes ratio molaires, à savoir dans un rapport fonctions NCO/fonctions COOH du monoester = 1 et ratio molaire fonctions NEt3 / fonctions COOH = 0,01.

Le produit obtenu est bien le produit de réaction attendu et présente les bandes caractéristiques majoritaires suivantes :

Absence de bande NCO à 2257$cm^{-1}$
Bande C=O carbonate à 1798 $cm^{-1}$
Bande C=O ester succinate à 1743 $cm^{-1}$
Bandes isocyanurates de l'HDT à 1687 et 1468 $cm^{-1}$
Bande -C=O-NH- amide à 1546 $cm^{-1}$ et Bande C=O amide secondaire à 1640 $cm^{-1}$
Bande NH à 3350 - 3250 $cm^{-1}$

## EXEMPLE 9 :

**Synthèse d'une résine à fonctions carbonates pendantes et à motifs essentiellement isocyanurates obtenue par réaction du composé de l'exemple 6 et du trimère isocyanurate de l'isophorone diisocyanate (IPDT)**

L'IPDT est un produit solide (Point de fusion 100-115°C), obtenu par cyclopolycondensation de l'isophorone diisocyanate (IPDI) sur lui même à motifs isocyanurates et fonctions isocyanates libres. Le titre en fonctions isocyanates est de 0,409 mole de fonctions NCO par 100g de produit.

On procède comme pour l'exemple précédent en travaillant sur 15,1 g d'IPDT solide de la société Creanova Huels.

On utilise les mêmes ratio molaires à savoir dans un rapport fonctions NCO / fonctions COOH du monoester = 1 et ratio molaire fonctions NEt3 / fonctions COOH = 0,01.

Le produit obtenu est bien le produit de réaction attendu mais il présente encore des fonctions isocyanates n'ayant pas réagi et des fonctions acides libres à raison de 20 % molaires des fonctions initiales et présente les bandes caractéristiques majoritaires suivantes :

Bande NCO à 2257cm$^{-1}$
Bande C=O carbonate à 1788 cm$^{-1}$
Bande C=O ester succinate à 1735 cm$^{-1}$
Bandes isocyanurates de l'IPDT à 1693 et 1446 cm$^{-1}$
Bande -C=O-NH- amide à 1546 cm$^{-1}$ et Bande C=O amide secondaire à 1640 cm$^{-1}$
Bande NH à 3350 - 3250 cm$^{-1}$
Fonction acide OH à 3200- 2500 cm$^{-1}$

**EXEMPLE 10 :**

**Synthèse d'une résine à fonctions carbonates pendantes, à fonctions isocyanates masquées par le 1,2,4-triazole et motifs essentiellement isocyanurates obtenue par réaction du composé de l'exemple 6, du 1,2,4 triazole et du Tolonate® HDT.**

On procède comme pour l'exemple précédent en utilisant 15 g de Tolonate® HDT (cf exemple 6), 2,797 g de 1,2,4-triazole et 8,77 g de monoester de carbonate de glycérol de l'acide succinique. Le ratio molaire fonctions COOH/ fonctions NCO est de 0,5, le ratio molaire fonctions triazole/fonctions NCO est de 0,5 et le ratio molaire fonctions $NEt_3$/ fonctions COOH est de 1%.

Le produit obtenu est bien le produit de réaction attendu et présente les bandes caractéristiques majoritaires suivantes :

Absence de bande NCO à 2257cm$^{-1}$
Bande C=O carbonate à 1788 cm$^{-1}$
Bande C=O ester succinate plus C=O bloqué par le triazole à 1739 cm$^{-1}$
Bandes isocyanurates de l'HDT à 1684 et 1467 cm$^{-1}$
Bande amides difficilement observables -C=O-NH- amide à 1546 cm$^{-1}$ et Bande C=O amide secondaire à 1640 cm$^{-1}$
Bandes du blocage par le triazole à 3337, 3126, 1531, 1506 cm$^{-1}$

Le produit obtenu présente donc 50 % des bandes isocyanates sous forme masquées temporairement par le 1,2,4-triazole qui peuvent être régénérées par effet thermique à une température de l'ordre de 130-140°C. Les 50 % molaires de fonctions isocyanates restantes ont été transformées en liaisons amides par réaction avec les fonctions acides et forment le lien entre la chaîne aliphatique ($(CH_2)_6$) portée par le motif isocyanurate et la chaîne succinylcarbonate.

Ce composé se caractérise donc par un ratio molaire NCO masqué par le 1,2, 4-triazole / fonctions carbonates égal à 1.

**EXEMPLE 11 :**

**Synthèse du monoester de carbonate de glycérol de l'acide glutarique**

On procède comme pour l'exemple 6 en remplaçant mole à mole l'anhydride succinique par l'anhydride glutarique.

On obtient le monoester de carbonate de glycérol et d'acide glutarique sous forme d'un composé solide avec un rendement de 52 %. Ce produit présente les bandes caractéristiques infra rouge identiques à celles du dérivé du composé de l'exemple 6.

**EXEMPLES 12-15 :**

**Synthèse d'une résine à fonctions carbonates pendantes et à motifs essentiellement isocyanurates obtenue par réaction du composé de l'exemple 6 et de polyisocyanates isocyanurates.**

On procède comme pour les exemples 7 à 10 en remplaçant mole à mole le composé de l'exemple 6 par le composé de l'exemple 11 (monoester du carbonate de glycérol et d'acide glutarique).

Les produits obtenus sont conformes aux produits attendus et sont confirmés par analyse infra rouge.

Les bandes caractéristiques majoritaires infra rouge sont celles respectivement indiquées pour les composés des exemples 7-10.

**EXEMPLE 16 :**

**Synthèse d'une résine à fonctions carbonates pendantes et à motifs essentiellement isocyanurates obtenue par réaction du Tolonate® HDT, du triméthylol propane et du carbonyl diimidazole.**

Dans un réacteur sous atmosphère inerte d'azote, on charge 1 mole de triméthylolpropane (134 g), 1mole de carbonyl diimidazole (165 g) et 100 g de Solvesso® 100. Le milieu réactionnel agité est chauffé à 90°C pendant 5 heures.

Au mélange réactionnel chaud, on ajoute 600 g de Tolonate® HDT et le milieu réactionnel est laissé sous agitation à 75°C pendant une nuit. Une analyse infra rouge du milieu réactionnel indique que la bande isocyanate a pratiquement disparu.

L'analyse du produit par infra rouge indique que l'on obtient bien le produit attendu à savoir une résine polyisocyanurate dont 2/ 3 environ des fonctions isocyanates sont bloquées par l'imidazole et 1/3 environ des fonctions isocyanates sont bloquées sous forme de carbamate du carbonate de triméthylolpropane.

On montre ainsi que l'on peut en une seule étape préparer une résine thermoréticulable en utilisant un composé carbonylé activé par des groupes partants qui peuvent être utilisés comme groupes protecteurs temporaires des fonctions isocyanates.

**EXEMPLE 17 :**

**Synthèse du carbonate de l'acide 9,10-dihydroxy stéarique**

Dans un réacteur on introduit 10 g d'acide 9,10-dihydroxy stéarique. On ajoute une quantité molaire de soude (1 N) normale pour neutraliser la totalité de la fonction carboxylique sous forme de sel de sodium. On ajoute 100 ml de N,N-diméthyl formamide et on agite à 80°C pendant une heure. On procède ensuite à l'évaporation sous vide de 90 % du solvant pour éliminer toute l'eau.

On ajoute ensuite 100ml de N,N-diméthyl formamide sec et 5,22 g de carbonyl diimidazole et on laisse sous agitation et sous courant d'azote à 80°C une nuit.

Le produit est ensuite concentré à sec à l'évaporateur rotatif sous vide pour éliminer 95 % du N,N-diméthylformamide.

On ajoute au produit une solution aqueuse acide pour transformer le sel de sodium en acide correspondant de telle manière que la valeur mesurée au papier pH soit de 2. Le carbonate acide est ensuite extrait trois fois par 180 ml de toluène.

Les phases organiques toluéniques sont rassemblées et le solvant évaporé sous vide pour obtenir 12,3 g de produit pâteux.

La structure du produit est confirmée par analyse RMN $^1$H (DMSO).

On confirme bien la présence de la fonction carbonate et de la fonction acide carboxylique.

**EXEMPLE 18 :**

**Synthèse d'une résine à fonctions carbonates pendantes et à motifs isocyanurates**

Dans un réacteur, on charge du Tolonate® HDT (0, 518 mole de fonction NCO pour 100 g de produit) et 12 g du carbonate de l'acide 9,10-dihydroxy stéarique, de telle manière que le ratio molaire fonctions NCO / fonctions COOH soit égal à 1. On ajoute 1% molaire de triéthylamine par rapport aux fonctions acides carboxyliques COOH. On laisse sous agitation et sous courant d'azote pendant 8 heures à 95°C.

Le produit obtenu est conforme au produit attendu. Il présente les bandes caractéristiques suivantes :

Pas de bande NCO à 2257 $cm^{-1}$
Bande C=O carbonate à 1788 $cm^{-1}$
Bande C=O ester succinate à 1739 $cm^{-1}$
Bandes isocyanurates de l'HDT à 1685 et 1466 $cm^{-1}$
Bande -C=O-NH- amide à 1546 $cm^{-1}$ et Bande C=O amide secondaire à 1640 $cm^{-1}$
Bande NH à 3350 - 3250 $cm^{-1}$

**EXEMPLE 19 :**

**Synthèse du 1,9,10-trihydroxy octadécane**

On utilise la procédure décrite dans le brevet US 2 443 280 du 15 juin 1948 en utilisant 100 g d'alcool oléique comme composé de départ. Le produit est récupéré avec un rendement de 25%.

**EXEMPLE 20 :**

**Synthèse d'une résine uréthane thermoréticulable à fonctions isocyanates masquées et à fonctions carbonates pendantes**

Dans un réacteur équipé d'un agitateur et sous atmosphère d'azote, on charge 0,1 mole du composé 1,9,10-trihydroxy octadécane de l'exemple 19 et 100 ml d'acétate de 2-(1-méthoxypropyle). 0,1 mole de Tolonate® HDT dont le titre NCO est de 0,518 mole pour 100 g est alors ajouté au milieu réactionnel. La température du milieu réactionnel est portée à une température comprise entre 45 et 50 °C. Après une heure de réaction sous agitation, on ajoute 0,12 mole de carbonyldi-imidazole. Le milieu réactionnel est agité à 50°C pendant 4 heures puis la température du milieu réactionnel est portée à environ 85°C et laissé sous agitation pendant une nuit.
Le milieu réactionnel est ensuite laissé à refroidir pour donner une composition visqueuse d'une résine uréthane qui se caractérise par l'absence de fonctions isocyanates libres et par la présence de fonctions isocyanates masquées par l'imidazole, fonctions carbamates, des fonctions carbonates majoritairement cycliques.
L'analyse infra rouge indique la présence des bandes caractéristiques suivantes :

Absence de bande NCO à 2257$cm^{-1}$
Bande C=O carbonate à 1785 $cm^{-1}$
Bandes carbamates (1720$cm^{-1}$) et blocage par l'imidazole vers 1735 $cm^{-1}$
Bandes isocyanurates de l'HDT à 1691 et 1467 $cm^{-1}$

**EXEMPLE 21 :**

**Préparation d'un prépolymère à fonctions isocyanates libres terminales**

Dans un réacteur tricol, on introduit successivement :

- 100 g de polyol aliphatique K-Flex 188 (polyester de King Industries) (6,97 % OH/100 g soit 0,41 mole OH/100 g) ;
- 1 680 g de HDI (5 moles).

On chauffe le milieu réactionnel à 80°C. On agite sous $N_2$ à 80°C pendant cinq heures, puis l'HDI en excès est éliminé par distillation sous vide poussé.
Le produit obtenu est un prépolymère uréthane à fonctions isocyanates terminales qui présente un titre NCO de 0,22 mole NCO pour 100 g donc 9,24 % NCO pour 100 g de produit.

**EXEMPLE 22 :**

**Synthèse d'un prépolymère à fonctions amino terminales**

150 g de prépolymère à fonctions NCO terminales de l'exemple 21 sont introduits dans un réacteur avec agitation et placé sous atmosphère inerte d'azote.
On ajoute 0,33 mole de chlorhydrate de 5-amino-1-pentanol et 600 g de toluène.
Le milieu réactionnel est chauffé à 60°C, en présence de 0,1 % de dibutyldilaurate d'étain, jusqu'à ce que le titre en NCO soit inférieur à 1 % (méthode de dosage des fonctions NCO par réaction à la dibutylamine et dosage de

l'amine résiduelle par l'HCl).

On obtient une solution toluénique visqueuse de polymère à fonctions chlorhydrates d'amine dont le titre en $NH_2$ est d'environ 0,04 mole $NH_2$/100 g.

## EXEMPLE 23 :

### Formation d'un revêtement

Dans un réacteur équipé d'un agitateur, on introduit 100g de la solution de polymère de l'exemple 22 (0,04 mole de fonctions amines sous forme chlorhydrate). On ajoute ensuite 20 g du produit de l'exemple 3 de telle manière que le ratio molaire fonctions amines/ fonctions O-C(=O)-O- soit égal à 1. On ajoute 0,04 mole de triéthylamine et 200g de toluène.

Le mélange est agité pendant 30 minutes à température ambiante.

On dépose le mélange obtenu sur une plaque de verre de telle manière à faire un film de 50 µm d'épaisseur puis on place à l'étuve à 50°C pendant 30 minutes puis 20 minutes à 100°C et 30 minutes à 140°C.

On obtient après cuisson un revêtement réticulé transparent qui présente de bonnes propriétés mécaniques.

Cet exemple montre que les produits de l'invention permettent d'obtenir des revêtements.

Cependant une optimisation des conditions de formulations (choix du solvant, mélange avec d'autres polyols ou d'autres amines, optimisation des ratio (NCO / OH), ( Amines / fonctions O-C(=O)-O-), nature du polyol acrylique ou polyester, nature des isocyanates engagés) permet d'adapter les propriétés des revêtements aux besoins recherchés.

## EXEMPLE 24 :

### Synthèse de l'HDT protégé par l'imidazole et le carbonate de triméthylolpropane

Dans un réacteur, on ajoute :

- 134 g de triméthylolpropane (1 mole) ;
- 1 mole de carbonyldiimidazole (165 g)

puis on chauffe la réaction à 80°C pendant cinq heures.

Au mélange réactionnel chaud, on ajoute 600 g de HDT et on laisse sous agitation à 80°C pendant cinq heures jusqu'à ce que la bande NCO disparaisse.

On obtient ainsi directement en une seule opération 900 g d'un dérivé de Tolonate® HDT dont 2/3 des fonctions NCO sont protégées par l'imidazole et 1/3 protégées par le carbonate de glycérol.

On montre ainsi qu'on peut obtenir ce dérivé en une seule opération en utilisant un dérivé carbonyle activé par des agents partants (imidazole, triazole, phényle) qui peuvent servir par la suite d'agents de masquage de la fonction NCO.

## Revendications

**1.** Composition pour application successive ou simultanée comprenant :

a) un (poly)isocyanate modifié stable dont au moins une des fonctions isocyanate est modifiée par un bras fonctionnel réticulant, ou groupe à fonctionnalité réticulante, ladite fonctionnalité réticulante étant un groupement carbonate cyclique ; et

b) un agent réactif à fonction amine primaire ou secondaire.

**2.** Composition selon la revendication 1, **caractérisée en ce que** la fonctionnalité réticulante carbonate cyclique est susceptible d'être formée par réaction de diols vicinaux, ou de triméthylolpropane, avec des agents de carbonylation activés.

**3.** Composition selon la revendication 2, **caractérisée en ce que** les diols vicinaux sont choisis parmi le glycérol, l'acide 9,10-dihydroxy stéarique et le 1,9,10-trihydroxy-octadécane.

**4.** Composition selon la revendication 2 ou la revendication 3, **caractérisée en ce que** les agents de carbonylation

activés sont choisis parmi le carbonyldiimidazole, le carbonyldi-1,2,4-triazole, le carbonyldiméthyléthylcétoxime et le N,N'-disuccinimidylcarbonate.

5. Composition selon la revendication 1 ou la revendication 2, **caractérisée en ce que** la fonctionnalité réticulante carbonate cyclique est choisie parmi le carbonate de glycérol, le monoester de carbonate de glycérol de l'acide succinique, le monoester de carbonate de glycérol de l'acide glutarique, le carbonate de triméthylolpropane, le carbonate de l'acide 9,10-dihydroxystéarique et le carbonate du 1,9,10-trihydroxyoctadécane.

6. Composition selon l'une quelconque des revendications précédentes, **caractérisée en ce que** le groupe à fonctionnalité réticulante résulte de la réaction d'une fonction isocyanate avec le carbonate de glycérol.

7. Composition selon la revendication 6, **caractérisée en ce que** le (poly)isocyanate modifié stable dont au moins une des fonctions isocyanate est modifiée par un bras fonctionnel réticulant, ou groupe à fonctionnalité réticulante, répond à la formule :

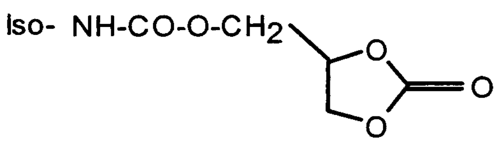

dans laquelle Iso représente le reste d'un (poly)isocyanate après transformation d'une fonction isocyanate.

8. Composition selon l'une des revendications 1 à 5, **caractérisée en ce que** le groupe à fonctionnalité réticulante résulte de la réaction d'une fonction isocyanate avec les carbonates d'acide gras ou leurs esters.

9. Composition selon la revendication 8, **caractérisée en ce que** le groupe à fonctionnalité réticulante résulte de la réaction d'une fonction isocyanate avec le 8,9-carbonate de l'acide oléique.

10. Composition selon l'une quelconque des revendications précédentes, **caractérisée en ce que** le (poly)isocyanate modifié stable a un poids moléculaire inférieur à 7500, avantageusement inférieur à 3500, de préférence inférieur à 2500.

11. Composition selon l'une quelconque des revendications précédentes, **caractérisée en ce que** le (poly)isocyanate modifié stable est choisi parmi :

- les diisocyanates ;
- les dérivés isocyanates, notamment polyisocyanates comportant un groupe isocyanurate encore appelés trimères ;
- les dérivés isocyanates, notamment polyisocyanates comprenant au moins un groupe urétidinedione, encore appelés dimères ;
- les dérivés isocyanates, notamment polyisocyanates comprenant au moins un groupe biuret ;
- les dérivés isocyanates, notamment polyisocyanates comprenant au moins un groupe carbamate ;
- les dérivés isocyanates, notamment polyisocyanates comprenant au moins un groupe allophanate ;
- les dérivés isocyanates, notamment polyisocyanates comprenant au moins un groupe ester ;
- les dérivés isocyanates, notamment polyisocyanates comprenant au moins un groupe amide ;
- les dérivés isocyanates notamment polyisocyanates comprenant au moins une fonction urée
- les dérivés isocyanates notamment polyisocyanates comprenant au moins une fonction imino-cylo-oxa-diazine-dione ;
- les dérivés isocyanates notamment polyisocyanates comprenant au moins une fonction cylo-oxa-diazine-trione ;
- les dérivés isocyanates, notamment polyisocyanates comprenant au moins un groupe isocyanate masqué ; et
- les dérivés isocyanates, notamment polyisocyanates comprenant une combinaison d'un ou plusieurs des groupes qui viennent d'être mentionnés, en particulier un groupe isocyanurate.

12. Composition selon l'une quelconque des revendications précédentes, **caractérisée en ce que** le (poly)isocyanate modifié stable comporte en outre au moins une autre fonction isocyanate libre et/ou au moins une autre fonction

isocyanate masquée par un agent masquant ou un mélange d'agents masquants thermolabiles.

**13.** Composition selon la revendication 12, **caractérisée en ce que** l'agent masquant est choisi parmi l'imidazole, le pyrazole, le 1,2,3-triazole et le 1,2,4-triazole, substitués ou non substitués.

**14.** Composition selon la revendication 12 ou la revendication 13, **caractérisée en ce que** les fonctions isocyanate non modifiées peuvent être masquées par aux moins deux groupes masquants différents.

**15.** Composition selon l'une quelconque des revendications 12 à 14, **caractérisée en ce que** le (poly)isocyanate modifié stable comporte au moins deux agents de masquage différents, choisis de manière que dans le test à l'octanol à 110°C, le rapport

$$D = \frac{\text{pourcentage d'agent masquant se débloquant en premier à 110°C}}{\text{pourcentage d'agent masquant se débloquant en dernier à 110°C}}$$

soit supérieur à 4/3, avantageusement supérieur à 1,5, de préférence supérieur à 2.

**16.** Composition selon la revendication 15, **caractérisée en ce que** les groupes masquants sont respectivement une oxime et le triazole (1,2,3-triazole ou 1,2,4-triazole), l'oxime étant avantageusement la méthyl-éthyl cétoxime, la méthyl-amyl-cétoxime, l'oxime du pyruvate de méthyle ou l'oxime du pyruvate d'éthyle.

**17.** Composition selon l'une quelconque des revendications précédentes, **caractérisée en ce que** l'agent réactif à fonction amine primaire ou secondaire est choisi parmi l'ammoniaque, les amines primaires ou secondaires ou leurs sels, et les hétérocycles azotés, dont les guanidines ou leurs sels qui agissent par ouverture de cycle.

**18.** Composition selon la revendication 17, **caractérisée** en ce l'agent réactif est un agent réactif à fonction amine primaire.

**19.** Composition selon la revendication 17, **caractérisée** en ce l'agent réactif est un agent réactif à fonction amine secondaire.

**20.** Composition selon l'une quelconque des revendications 17 à 19, **caractérisée** en ce l'agent réactif à fonction aminé primaire ou secondaire est une diamine ou une polyamine.

**21.** Composition selon l'une quelconque des revendications 17 à 19, **caractérisée en ce que** l'agent réactif à fonction amine primaire ou secondaire est une amine polyalcoxylée ou une amine silicone ou une amine perfluorée.

**22.** Procédé de génération d'une liaison carbamate dans un (poly)isocyanate modifié stable dont au moins une des fonctions isocyanate est modifiée par un bras fonctionnel réticulant, ou groupe à fonctionnalité réticulante, ladite fonctionnalité réticulante étant un groupement carbonate cyclique, par réaction d'ouverture du cycle carbonate avec un agent réactif à fonction amine primaire ou secondaire.

**23.** Procédé selon la revendication 22, **caractérisé en ce que** la fonctionnalité réticulante carbonate cyclique est telle que définie dans l'une quelconque des revendications 2 à 9.

**24.** Procédé selon la revendication 22, **caractérisé en ce que** le (poly)isocyanate modifié stable est tel que défini dans l'une quelconque des revendications 10 à 16.

**25.** Procédé selon la revendication 22, **caractérisé en ce que** l'agent réactif à fonction amine primaire ou secondaire est tel que défini dans l'une quelconque des revendications 17 à 21.

**26.** Utilisation d'une composition selon l'une quelconque des revendications 1 à 21 pour la préparation de revêtements minces non expansés, notamment de peintures ou vernis.

Office européen des brevets

# RAPPORT DE RECHERCHE EUROPEENNE

Numéro de la demande
EP 03 02 2382

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | EP 0 395 122 A (HOECHST AG)<br>31 octobre 1990 (1990-10-31)<br>* page 5, ligne 25 - page 6, ligne 50 *<br>* exemples III,VI *<br>--- | 1,10-12, 17,18, 20,22, 24-26 | C08G18/80<br>C09D175/02 |
| A | CHEMICAL ABSTRACTS, vol. 119, no. 20, 15 novembre 1993 (1993-11-15)<br>Columbus, Ohio, US;<br>abstract no. 205580,<br>XP002123895<br>* abrégé *<br>& JP 50 981168 A (DAINIPPON INK & CHEMICALS)<br>--- | 1-26 | |
| A,D | EP 0 337 926 A (DAINIPPON INK & CHEMICALS)<br>18 octobre 1989 (1989-10-18)<br>* page 2, ligne 48 - page 4, ligne 43 *<br>* exemple 4 *<br>* revendications 3,9 *<br>----- | 1-26 | |

DOMAINES TECHNIQUES RECHERCHES (Int.Cl.7)
C08G
C09D

Le présent rapport a été établi pour toutes les revendications

| Lieu de la recherche | Date d'achèvement de la recherche | Examinateur |
|---|---|---|
| MUNICH | 24 novembre 2003 | Neugebauer, U |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : *document intercalaire*

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 03 02 2382

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

24-11-2003

| Document brevet cité au rapport de recherche | Date de publication | Membre(s) de la famille de brevet(s) | Date de publication |
|---|---|---|---|
| EP 0395122 A | 31-10-1990 | DE 3624454 A1 | 20-08-1987 |
| | | AT 60790 T | 15-02-1991 |
| | | AT 139553 T | 15-07-1996 |
| | | AU 598231 B2 | 21-06-1990 |
| | | AU 6872787 A | 20-08-1987 |
| | | BR 8700663 A | 15-12-1987 |
| | | CA 1335735 C | 30-05-1995 |
| | | CN 87100667 A ,B | 26-08-1987 |
| | | DE 3751844 D1 | 25-07-1996 |
| | | DE 3767891 D1 | 14-03-1991 |
| | | EP 0234395 A2 | 02-09-1987 |
| | | EP 0395122 A2 | 31-10-1990 |
| | | ES 2090060 T3 | 16-10-1996 |
| | | FI 870582 A ,B, | 14-08-1987 |
| | | GR 3001467 T3 | 08-10-1992 |
| | | GR 3020291 T3 | 30-09-1996 |
| | | JP 2716433 B2 | 18-02-1998 |
| | | JP 62192348 A | 22-08-1987 |
| | | JP 2872651 B2 | 17-03-1999 |
| | | JP 10101998 A | 21-04-1998 |
| | | JP 2872652 B2 | 17-03-1999 |
| | | JP 10101769 A | 21-04-1998 |
| | | KR 9505505 B1 | 24-05-1995 |
| | | MX 5225 A ,B | 01-06-1993 |
| | | US 4808658 A | 28-02-1989 |
| | | ZA 8701007 A | 30-09-1987 |
| JP 50981168 A | | AUCUN | |
| EP 0337926 A | 18-10-1989 | DE 3809840 A1 | 05-10-1989 |
| | | EP 0337926 A2 | 18-10-1989 |
| | | JP 2002866 A | 08-01-1990 |
| | | JP 3041856 B2 | 15-05-2000 |

EPO FORM P0460

Pour tout renseignement concernant cette annexe : voir Journal Officiel de l'Office européen des brevets, No.12/82